# EUROPEAN PATENT APPLICATION

(11) **EP 2 939 669 A1**
(43) Date of publication of application: **04.11.2015**
(21) Application number: 15169481.7
(22) Date of filing: 21.12.2010
(51) Int. Cl.: A61K 31/135, C07C 225/20, C07C 221/00

(54) **3-KETO-N-PROPARGYL-1-AMINOINDAN**

(30) Priority: 22.12.2009 US 284757 P; 15.10.2010 US 393771 P
(62) Divisional of application: 10843543.9
(71) Applicant: TEVA PHARMACEUTICAL INDUSTRIES, LTD., 49131 Petah Tiqva (IL)
(72) Inventor: Frenkel, Anton, 42202 Netanya (IL); Lidor-Hadas, Ramy, 44242 Kfar-Saba (IL); Bahar, Eliezer, 69345 Tel-Aviv (IL)
(74) Representative: D Young & Co LLP

(57) **Abstract**

The subject invention provides a pharmaceutical composition containing N-propargyl-1(R)-aminoindan or a pharmaceutically acceptable salt thereof, and a compound of 3-keto-N-propargyl-1-aminoindan or a salt thereof.

## Description

This application claims priority of U.S. Provisional Applications Nos. 61/393,771, filed October 15, 2010 and 61/284,757, filed December 22, 2009, the contents of each of which are hereby incorporated by reference.

Throughout this application various publications, published patent applications, and patents are referenced. The disclosures of these documents in their entireties are hereby incorporated by reference into this application in order to more fully describe the state of the art to which this invention pertains.

### Background of the Invention

United States Patents 5,532,415, 5,387,612, 5,453,446, 5,457,133, 5,599,991, 5,744,500, 5,891,923, 5,668,181, 5,576,353, 5,519,061, 5,786,390, 6,316,504, 6,630,514 disclose R(+)-N-propargyl-1-aminoindan ("R-PAI"), also known as rasagiline. Rasagiline has been reported to be a selective inhibitor of the B-form of the enzyme monoamine oxidase ("MAO-B") and is useful in treating Parkinson's disease and various other conditions by inhibition of MAO-B in the brain.

United States Patents 6,126,968, 7,572,834, 7,598,420, United States Patent applications 12/283,022, and 12/283,107 and PCT publications WO 95/11016 and WO 2006/014973, hereby incorporated by reference, disclose pharmaceutical compositions comprising rasagiline and processes for their preparation.

AZILECT^{®} is a commercially available rasagiline mesylate immediate release formulation indicated for the treatment of the signs and symptoms of idiopathic Parkinson's disease as initial monotherapy and as adjunct therapy to levodopa. The current marketed formulation of rasagiline (Azilect®) is rapidly absorbed, reaching peak plasma concentration (tₘₐₓ) in approximately 1 hour. The absolute bioavailability of rasagiline is about 36%. (AZILECT® Product Label, May 2006).

United States publication US 2008/161408 hereby incorporated by reference, discloses crystalline rasagiline base.

United States publication US 2009/0181086 and United States Patent applications 12/456,029, 12/456,031, 12/455,976 and 12/456,001, hereby incorporated by reference, disclose delayed release rasagiline formulations.

### Summary of the Invention

The subject invention provides a composition comprising N-propargyl-1(R)-aminoind or a pharmaceutically acceptable salt thereof, and 3-keto-N-propargyl-1-aminoind or a salt thereof, wherein the total amount of 3-keto-N-propargyl-1-aminoindan which is present in the composition is less than 0.10% relative to the amount of N-propargyl-1(R)-aminoindan, based on a determination by an HPLC method.

The subject invention also provides a process for the manufacture of a composition comprising N-propargyl-1(R)-aminoindan or a pharmaceutically acceptable salt thereof, comprising producing dry rasagiline tartrate from racemic propargyl aminoindan in metal-free equipment, and producing the composition.

The subject invention further provides a process for preparing a pharmaceutical product comprising N-propargyl-1(R)-aminoindan or a pharmaceutically acceptable salt thereof, and at least one pharmaceutically acceptable carrier, comprising:
a) obtaining a batch of N-propargyl-1(R)-aminoindan or a pharmaceutically acceptable salt thereof;
b) determining the total amount of 3-keto-N-propargyl-1-aminoindan in the batch; and
c) preparing the pharmaceutical product from the batch only if the batch is determined to have less than 0.10% 3-keto-N-propargyl-1-aminoind relative to N-propargyl-1(R)-aminoindan, based on a determination by an HPLC method.

The subject invention yet further provides a process of distributing a validated batch of a pharmaceutical product comprising N-propargyl-1(R)-aminoindan or a pharmaceutically acceptable salt thereof and at least one pharmaceutically acceptable carrier, comprising:
a) producing a batch of the pharmaceutical product;
b) performing stability testing with a sample of the batch;
c) determining the total amount of 3-keto-N-propargyl-1-aminoindan in the sample of the batch after stability testing; and
d) validating the batch for distribution only if the sample of the batch after stability testing is determined to have less than 0.10% of 3-keto-N-propargyl-1-aminoind relative to N-propargyl-1(R)-aminoindan, based on a determination by an HPLC method.

The subject invention yet further provides an isolated compound having the structure: 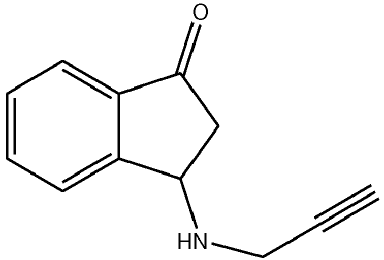 or a salt thereof.

The subject invention yet further provides a composition comprising a compound having the structure: 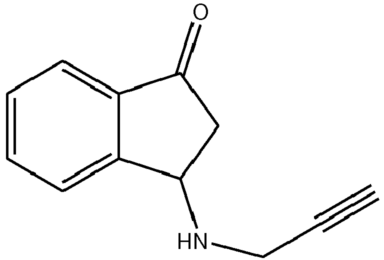 wherein the composition is free of N-propargyl-1-aminoindan or a salt thereof.

The subject invention yet further provides a process for the manufacture of 3-keto-N-propargyl-1-aminoindan, or an enantiomer or a salt thereof, comprising reacting 1-aminoindane-3-one with a propargylating agent in the presence of a base so as to produce the compound.

The subject invention yet further provides a use of 3-keto-N-propargyl-1-aminoinda or an enantiomer or a salt thereof, as a reference standard to detect trace amounts of impurities in a pharmaceutical product comprising N-propargyl-1(R)-aminoindan or a pharmaceutically acceptable salt thereof.

### Brief Description of the Figures

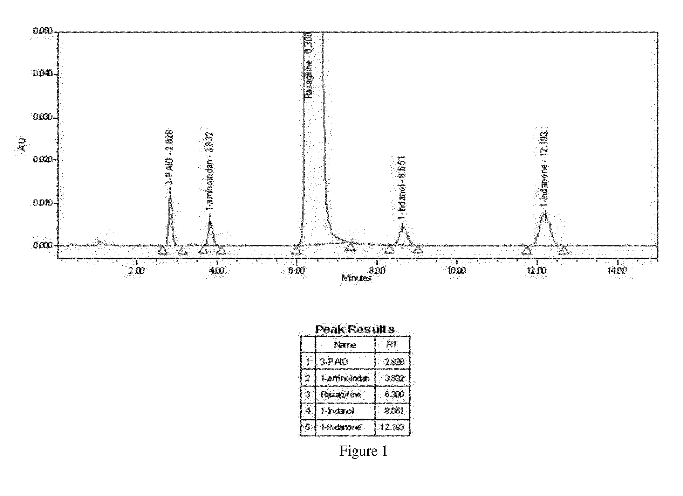

Figure 1 shows retention times of rasagiline and its impurities.

### Detailed Description of the Invention

The subject invention provides a composition comprising N-propargyl-1(R)-aminoind or a pharmaceutically acceptable salt thereof, and 3-keto-N-propargyl-1-aminoind or a salt thereof, wherein the total amount of 3-keto-N-propargyl-1-aminoindan which is present in the composition is less than 0.10% relative to the amount of N-propargyl-1(R)-aminoindan, based on a determination by an HPLC method.

In an embodiment of the composition, the total amount of 3-keto-N-propargyl-1-aminoindan which is present in the composition is greater than 0.02% relative to the amount of N-propargyl-1(R)-aminoindan.

In another embodiment of the composition, the total amount of 3-keto-N-propargyl-1-aminoindan which is present in the composition is greater than 0.05% relative to the amount of N-propargyl-1(R)-aminoindan.

In yet another embodiment of the composition, the total amount of 3-keto-N-propargyl-1-aminoindan which is present in the composition is less than 0.05% relative to the amount of N-propargyl-1(R)-aminoindan.

In yet another embodiment of the composition, the total amount of 3-keto-N-propargyl-1-aminoindan which is present in the composition is less than 0.02% relative to the amount of N-propargyl-1(R)-aminoindan.

In yet another embodiment of the composition, the pharmaceutically acceptable salt of N-propargyl-1(R)-aminoindan is a mesylate salt.

In yet another embodiment of the composition, the pharmaceutically acceptable salt of N-propargyl-1(R)-aminoindan is a citrate salt.

In yet another embodiment of the composition, N-propargyl-1(R)-aminoindan is present in the form of a free base.

In yet another embodiment of the composition, the composition further comprises at least one pharmaceutically acceptable carrier.

In yet another embodiment of the composition, the pharmaceutically acceptable carrier is selected from the group consisting of mannitol, starch, pregelatinized starch, colloidal silicon dioxide, stearic acid and talc.

In yet another embodiment of the composition, the 3-keto-N-propargyl-1-aminoind is 3-keto-N-propargyl-1(R)-aminoindan.

The subject invention also provides a process for the manufacture of a composition comprising N-propargyl-1(R)-aminoindan or a pharmaceutically acceptable salt thereof, comprising producing dry rasagiline tartrate from racemic propargyl aminoindan in metal-free equipment, and producing the composition.

In an embodiment of the process, the step of producing dry rasagiline tartrate from racemic propargyl aminoindan is performed under an inert atmosphere.

In another embodiment of the process, the pharmaceutically acceptable salt of N-propargyl-1(R)-aminoindan is a mesylate salt.

In yet another embodiment of the process, the pharmaceutically acceptable salt of N-propargyl-1(R)-aminoindan is a citrate salt.

In yet another embodiment of the process, N-propargyl-1(R)-aminoindan is present in the form of a free base.

In yet another embodiment of the process, the inert atmosphere is a nitrogen atmosphere.

In yet another embodiment of the process, the metal-free equipment is glassware equipment.

The subject invention further provides a process for preparing a pharmaceutical product comprising N-propargyl-1(R)-aminoindan or a pharmaceutically acceptable salt thereof, and at least one pharmaceutically acceptable carrier, comprising:
d) obtaining a batch of N-propargyl-1(R)-aminoindan or a pharmaceutically acceptable salt thereof;
e) determining the total amount of 3-keto-N-propargyl-1-aminoindan in the batch; and
f) preparing the pharmaceutical product from the batch only if the batch is determined to have less than 0.10% 3-keto-N-propargyl-1-aminoind relative to N-propargyl-1(R)-aminoindan, based on a determination by an HPLC method.

In an embodiment of the process, in step c) the pharmaceutical product is prepared from the batch only if the batch is determined to have 3-keto-N-propargyl-1-aminoind present in an amount of less than 0.10% relative to N-propargyl-1(R)-aminoindan.

In another embodiment of the process, the pharmaceutical product is prepared from the batch only if the batch is determined to have less than 0.05% 3-keto-N-propargyl-1-aminoind relative to N-propargyl-1(R)-aminoindan.

In yet another embodiment of the process, the pharmaceutical product is prepared from the batch only if the batch is determined to have less than 0.02% 3-keto-N-propargyl-1-aminoindan relative to N-propargyl-1(R)-aminoindan.

In yet another embodiment of the process, the pharmaceutical product is prepared from the batch if the batch is determined to have 3-keto-N-propargyl-1-aminoind present in an amount of greater than 0.05% relative to N-propargyl-1(R)-aminoindan.

In yet another embodiment of the process, the pharmaceutical product is prepared from the batch if the batch is determined to have 3-keto-N-propargyl-1-aminoind present in an amount of greater than 0.02% relative to N-propargyl-1-aminoindan.

In yet another embodiment of the process, the pharmaceutically acceptable salt of N-propargyl-1(R)-aminoindan is a mesylate salt.

In yet another embodiment of the process, the pharmaceutically acceptable salt of N-propargyl-1(R)-aminoindan is a citrate salt.

In yet another embodiment of the process, N-propargyl-1(R)-aminoindan is present in the form of a free base.

The subject invention yet further provides a process of distributing a validated batch of a pharmaceutical product comprising N-propargyl-1(R)-aminoindan or a pharmaceutically acceptable salt thereof and at least one pharmaceutically acceptable carrier, comprising:
a) producing a batch of the pharmaceutical product;
b) performing stability testing with a sample of the batch;
c) determining the total amount of 3-keto-N-propargyl-1-aminoindan in the sample of the batch after stability testing; and
d) validating the batch for distribution only if the sample of the batch after stability testing is determined to have less than 0.10% of 3-keto-N-propargyl-1-aminoind relative to N-propargyl-1(R)-aminoindan, based on a determination by an HPLC method.

In an embodiment of the process, in step d) the batch is validated only if the sample of the batch after stability testing is determined to have 3-keto-N-propargyl-1-aminoindan present in an amount of less than 0.10% of relative to N-propargyl-1(R)-aminoindan.

In another embodiment of the process, the batch is validated only if the sample of the batch after the stability testing is determined to have less than 0.05% 3-keto-N-propargyl-1-aminoindan relative to N-propargyl-1(R)-aminoindan.

In yet another embodiment of the process, the batch is validated only if the sample of the batch after the stability testing is determined to have less than 0.02% 3-keto-N-propargyl-1-aminoindan relative to N-propargyl-1(R)-aminoindan.

In yet another embodiment of the process, the batch is validated if the sample of the batch after the stability testing is determined to have 3-keto-N-propargyl-1-aminoindan present in an amount of greater than 0.02% of relative to N-propargyl-1(R)-aminoindan.

In yet another embodiment of the process, the batch is validated if the sample of the batch after the stability testing is determined to have 3-keto-N-propargyl-1-aminoind present in an amount of greater than 0.05% of relative to N-propargyl-1(R)-aminoindan.

In yet another embodiment of the process, the pharmaceutically acceptable salt of N-propargyl-1(R)-aminoindan is a mesylate salt.

In yet another embodiment of the process, the pharmaceutically acceptable salt of N-propargyl-1(R)-aminoindan is a citrate salt.

In yet another embodiment of the process, N-propargyl-1(R)-aminoindan is present in the form of a free base.

In yet another embodiment of the process, the pharmaceutically acceptable carrier is selected from the group consisting of mannitol, starch, pregelatinized starch, colloidal silicon dioxide, stearic acid and talc.

In yet another embodiment of the process, 3-keto-N-propargyl-1-aminoindan is 3-keto-N-propargyl-1(R)-aminoindan.

The subject invention yet further provides an isolated compound having the structure: 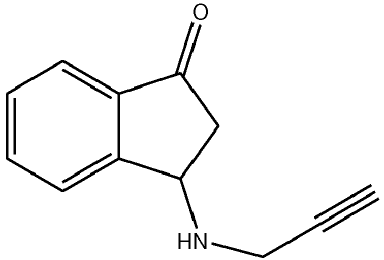 or a salt thereof.

In an embodiment of the isolated compound, the compound has the structure:

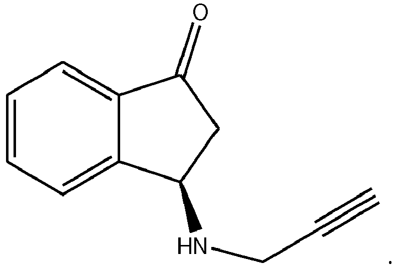

In another embodiment of the isolated compound, the compound has the structure:

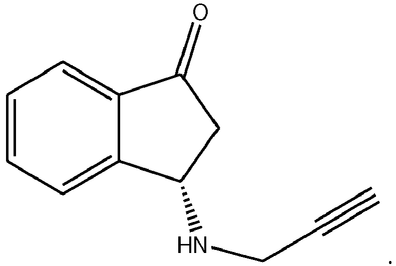

The subject invention yet further provides a composition comprising a compound having the structure: 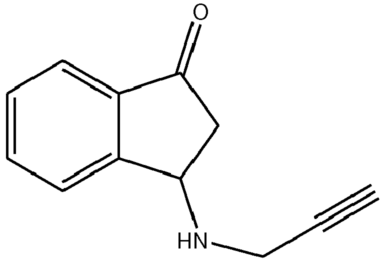 wherein the composition is free of N-propargyl-1-aminoindan or a salt thereof.

In an embodiment of the composition, the compound has the structure:

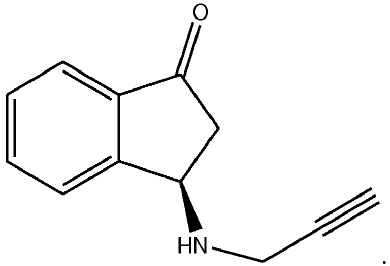

In another embodiment of the composition, the compound has the structure:

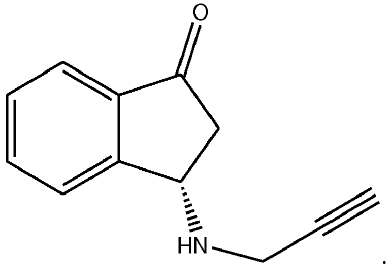

The subject invention yet further provides a process for the manufacture of 3-keto-N-propargyl-1-aminoindan, or an enantiomer or a salt thereof, comprising reacting 1-aminoindane-3-one with a propargylating agent in the presence of a base so as to produce the compound.

In an embodiment of the process, the process further comprises a step of purifying the 3-keto-N-propargyl-1(R)-aminoindan enantiomer.

In another embodiment of the process, 1-aminoindane-3-one is in the form of a hydrochloride salt.

In yet another embodiment of the process, the propargylating agent is selected from the group consisting of propargyl-bromide, propargyl-chloride, propargyl-iodide and propargyl alkyl sulfonate.

The subject invention yet further provides a use of 3-keto-N-propargyl-1-aminoinda or an enantiomer or a salt thereof, as a reference standard to detect trace amounts of impurities in a pharmaceutical product comprising N-propargyl-1(R)-aminoindan or a pharmaceutically acceptable salt thereof.

In an embodiment of the use, the enantiomer of 3-keto-N-propargyl-1-aminoind is 3-keto-N-propargyl-1(R)-aminoindan.

In another embodiment of the use, the impurity is a by-product.

It will be noted that the structure of the compounds of this invention includes an asymmetric carbon atom and thus the compounds occur as racemates, racemic mixtures, and isolated single enantiomers. All such isomeric forms of these compounds are expressly included in this invention. Each stereogenic carbon may be of the R or S configuration. It is to be understood accordingly that the isomers arising from such asymmetry (e.g., all enantiomers and diastereomers) are included within the scope of this invention, unless indicated otherwise. Such isomers can be obtained in substantially pure form by classical separation techniques and by stereochemically controlled synthesis, such as those described in "Enantiomers, Racemates and Resolutions" by J. Jacques, A. Collet and S. Wilen, Pub. John Wiley & Sons, NY, 1981. For example, the resolution may be carried out by preparative chromatography on a chiral column.

The subject invention is also intended to include all isotopes of atoms occurring on the compounds disclosed herein. Isotopes include those atoms having the same atomic number but different mass numbers. By way of general example and without limitation, isotopes of hydrogen include tritium and deuterium. Isotopes of carbon include C-13 and C-14.

It will be noted that any notation of a carbon in structures throughout this application, when used without further notation, are intended to represent all isotopes of carbon, such as ¹²C, ¹³C, or ¹⁴C. Furthermore, any compounds containing ¹³C or ¹⁴C may specifically have the structure of any of the compounds disclosed herein.

It will also be noted that any notation of a hydrogen in structures throughout this application, when used without further notation, are intended to represent all isotopes of hydrogen, such as ¹H, ²H, or ³H. Furthermore, any compounds containing ²H or ³H may specifically have the structure of any of the compounds disclosed herein.

Isotopically-labeled compounds can generally be prepared by conventional techniques known to those skilled in the art or by processes analogous to those described in the Examples disclosed herein using an appropriate isotopically-labeled reagents in place of the non-labeled reagents employed.

A characteristic of a compound refers to any quality that a compound exhibits, e.g., peaks or retention times, as determined by 1H nuclear magnetic spectroscopy, mass spectroscopy, infrared, ultraviolet or fluorescence spectrophotometry, gas chromatography, thin layer chromatography, high performance liquid chromatography, elemental analysis, Ames test, dissolution, stability and any other quality that can be determined by an analytical method. Once the characteristics of a compound are known, the information can be used to, for example, screen or test for the presence of the compound in a sample.

As used herein, a "pharmaceutically acceptable" carrier or excipient is one that is suitable for use with humans and/or animals without undue adverse side effects (such as toxicity, irritation, and allergic response) commensurate with a reasonable benefit/risk ratio.

Specific examples of pharmaceutical acceptable carriers and excipients that may be used to formulate oral dosage forms are described, e.g., in U.S. Pat. No. 6,126,968 to Peskin et al., issued Oct. 3, 2000. Techniques and compositions for making dosage forms useful in the present invention are described-in the following references: 7 Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modern Pharmaceutics Drugs and the Pharmaceutical Sciences, Vol 40 (Gilbert S. Banker, Christopher T. Rhodes, Eds.).

Tablets may contain suitable binders, lubricants, disintegrating agents, coloring agents, flavoring agents, flow-inducing agents, melting agents, stabilizing agents, solubilizing agents, antioxidants, buffering agent, chelating agents, fillers and plasticizers. For instance, for oral administration in the dosage unit form of a tablet or capsule, the active drug component can be combined with an oral, non-toxic, pharmaceutically acceptable, inert carrier such as gelatin, agar, starch, methyl cellulose, dicalcium phosphate, calcium sulfate, mannitol, sorbitol, microcrystalline cellulose and the like. Suitable binders include starch, gelatin, natural sugars such as corn starch, natural and synthetic gums such as acacia, tragacanth, or sodium alginate, povidone, carboxymethylcellulose, polyethylene glycol, waxes, and the like. Antioxidants include ascorbic acid, fumaric acid, citric acid, malic acid, gallic acid and its salts and esters, butylated hydroxyanisole, editic acid. Lubricants used in these dosage forms include sodium oleate, sodium stearate, sodium benzoate, sodium acetate, stearic acid, sodium stearyl fumarate, talc and the like. Disintegrators include, without limitation, starch, methyl cellulose, agar, bentonite, xanthan gum, croscarmellose sodium, sodium starch glycolate and the like, suitable plasticizers include triacetin, triethyl citrate, dibutyl sebacate, polyethylene glycol and the like.

As used herein, "drug substance" refers to the active ingredient in a drug product, which provides pharmacological activity or other direct effect in the diagnosis, cure, mitigation, treatment, or prevention of disease, or to affect the structure or any function of the body of man or animals.

As used herein, "drug product" refers to the finished dosage form containing the drug substance as well as at least one pharmaceutically acceptable carrier.

As used herein, an "isolated" compound is a compound isolated from the crude reaction mixture following an affirmative act of isolation. The act of isolation necessarily involves separating the compound from the other known components of the crude reaction mixture, with some impurities, unknown side products and residual amounts of the other known components of the crude reaction mixture permitted to remain. Purification is an example of an affirmative act of isolation.

As used herein, a composition that is "free" of a chemical entity means that the composition contains, if at all, an amount of the chemical entity which cannot be avoided following an affirmative act intended to separate the chemical entity and the composition.

As used herein, "stability testing" refers to tests conducted at specific time intervals and various environmental conditions (e.g., temperature and humidity) to see if and to what extent a drug product degrades over its designated shelf life time. The specific conditions and time of the tests are such that they accelerate the conditions the drug product is expected to encounter over its shelf life. For example, detailed requirements of stability testing for finished pharmaceuticals are codified in 21 C.F.R §211.166, the entire content of which is hereby incorporated by reference.

Propargylated aminoindan refers to a compound having an aminoindan moiety with a propargyl substituent on the nitrogen atom, whether or not there exist any other substituents.

Specific salts provided by this invention are the mesylate, maleate, fumarate, tartrate, hydrochloride, hydrobromide, esylate, p-toluenesulfonate, benzoate, acetate, phosphate and sulfate salts.

For the preparation of pharmaceutically acceptable acid addition salts of the compounds of the invention, the free base can be reacted with the desired acids in the presence of a suitable solvent by conventional methods. Similarly, an acid addition salt may be converted to the free base form in a known manner.

U.S. Patent No. 6,126,968, the entire contents of which are incorporated herein by reference, disclosed that the stability of formulations comprising PAI can be significantly improved by the incorporation of relatively large amounts of certain alcohols. In particular, the alcohol is selected from the group of pentahydric or hexahydric alcohols (U.S. Patent No. 6,126,968). The alcohol is typically selected from mannitol, xylitol or sorbitol (U.S. Patent No. 6,126,968). The composition may further comprise citric acid (U.S. Patent No. 6,126,968).

(R)-PAI itself may be prepared, for example, according to the process described in Example 6B of WO 95/11016.

### Experimental Details:

### Example 1: Preparation of racemic 1-proparylaminoindan-3-one (3-PAIO) Mesylate salt

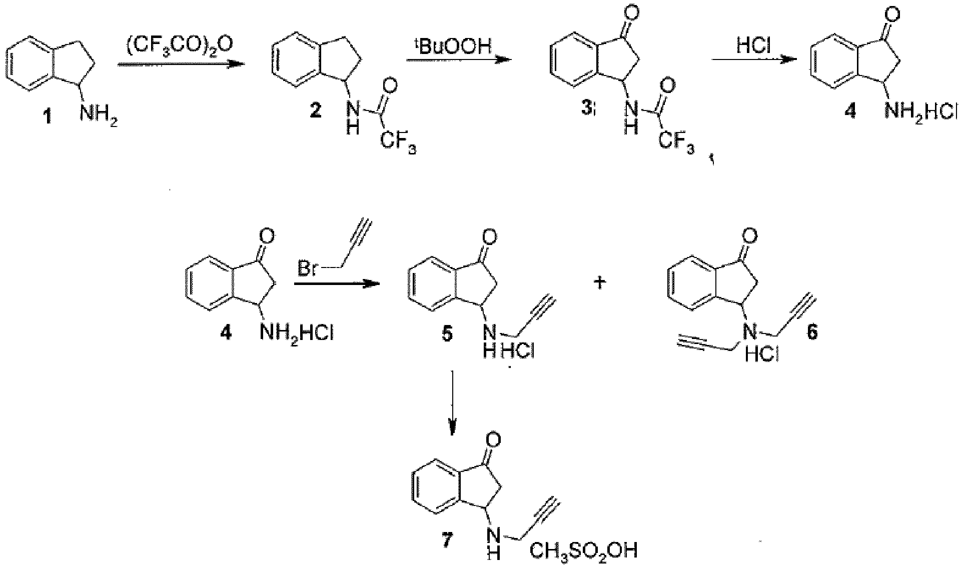

### Preparation of R-1-aminoindan hydrochloride 2

R-1-acetylaminoindan 1 (52.6 g; 0.30 mole) was added to a mixture of ethanol (600 ml) and concentrated hydrochloric acid (120 ml). The reaction mixture was boiled for 33 hours. At the end of the twentieth hour 30 ml of concentrated HCI was added. The reaction mixture was evaporated to dryness under reduced pressure. The residue (about 51 g) was boiled in acetone (560 ml) for ten minutes. The insoluble solid was collected by filtration of the hot suspension, washed with acetone, dried to give the first crop. The filtrate was evaporated to dryness in vacuum. 14.5 g of the starting material was recovered. This substance was subjected to hydrolysis according to the above to afford the second crop.
First crop: 35.1 g (69.0%), Mp: 233-236°C
Second crop: 9.8 g (19.2%), Mp: 224-230°C
Total: 44.9 g (88.2%)

### Preparation of R-1-trifluoroacetylaminoindan 3

A solution of potassium hydroxide (13.5 g; 0.24 mole) in water (40 ml) was added to R-1-aminoindan hydrochloride 2 (33.93 g; 0.20 mole) in water (60 ml). The mixture was stirred for 15 minutes (pH: 10-11), extracted with toluene (2 x 60 ml). The combined organic phase was dried on MgSO₄.

The solution of R-1-aminoindan base was added to a cooled (0-5°C), mixture of trifluoroacetic anhydride (50.4 g; 34 ml; 0.24 mole) and toluene (60 ml) over a period of 20 minutes. The reaction mixture was stirred at 0-5°C for 3.5 hours. A solution of KOH (17.5 g; 0.312 mole) in water (220 ml) was added to the reaction mixture at 0-5°C over a period of half an hour. The reaction mixture was stirred at room temperature for 2 hours. The solid was collected by filtration, washed with water. The second crop was obtained by separating the two phases of the mother liquid subsequently by evaporation of the organic phase to dryness.
First crop: 24.32 g (53.0%), Mp: 154.2 - 155.4°C
Second crop: 8.00 g (17.5%), Map: 150.4 -151.0°C
Total: 32.32 g (70.5%)

### R-1-triflaroroacetylaminoindan-3-one 4

A mixture of R-1-trifluoroacetylaminoindan **3** (22.92 g; 0.10 mole) and CrO₃ (0.50 g; 0.005 mole) in dichloromethane (200 ml) was cooled to 15°C. Tert-butyl hydroperoxide (70% solution in water; 95.5 ml; 0.69 mole) was added drop by drop at 24-26°C over a period of an hour. The reaction mixture was stirred overnight. The phases were separated. The aqueous phase was extracted with dichloromethane (200 ml). The combined organic phase was treated with a mixture of charcoal (2.6 g) and Al₂O₃ (6.6 g). The filtrate was evaporated to dryness. The residue was dissolved in EtOAc (120 ml) at 45°C, treated with charcoal (1.0 g), hexane (150 ml) was added, put into the fridge overnight. The solid was collected by filtration, washed with hexane. The mother liquid was treated with charcoal (0.40 g), evaporated to a very thick slurry. The solid was collected by filtration, washed with a small' amount of cold EtOAc then hexane to give the second crop.
First crop: 11.55 g (47.5%), Mp: 165.5-166.1°C
Second crop: 4.86 g (20.0%), Mp: 155.0-160.3°C
Total: 16.41 g (67.5%)

### R-1-aminoindan-3-one hydrochloride 5

A mixture of R-1-trifluoroacetylaminoindan-3-one **4** (24.32 g; 0.10 mole) and 6 N hydrochloric acid (360 ml) was refluxed for 45 minutes (TLC). The mixture was cooled to 5-10°C. The crystals were collected by filtration, washed with a small amount of cold EtOH, dried. The crude product was dissolved in water (about 140 ml) at room temperature, treated with charcoal (1.4 g), evaporated to a small volume under reduced pressure. The solid was collected by filtration, washed with cold acetone or/and EtOH. The mother liquids, derived from the reaction mixture and the purification step, were combined, treated with charcoal (0.60 g), evaporated to practically dryness. Acetone was added to the residue. The second crop was collected by filtration, washed with acetone then a small amount of EtOH.
First crop: 9.04 g (49.2%), Mp: 272-276°C
Second crop: 6.48 g (35.3%), Mp: 269-276°C
Total: 15.52 g (84.5%)

### Preparation of 1-Propargylaminoindan-3-one hydrochloride 5

To a mixture of potassium carbonate (34.55 g; 0.25 mole), acetonitrile (80 ml) and racemic 1-aminoindan-3-one hydrochloride **4** (18.36 g; 0.10 mole), propargyl bromide (80% in toluene; 17.8 g; 16.8 ml; 0.15 mole) was added over a period of 30 minutes at room temperature. The reaction mixture was stirred at room temperature for 18 hours. The mixture of the inorganic salts was collected by filtration. The filtrate was evaporated to dryness. The residue was then dissolved in acetone (200 ml) and 3.67 N HCl/EtOH (27.2 ml) was added. The mixture was put into the fridge overnight.

The crystals were collected by filtration, washed with acetone, dried. The crude product (17.3 g) was a mixture of 1-propargylaminoindan-3-one hydrochloride **5** and 1-dipropargylaminoindan-3-one hydrochloride **6.** The mixture of the salts was dissolved in a mixture of CHCl₃ (100 ml) and water (100 ml). After shaking the two phases were separated. The aqueous phase was extracted with CHCl₃ (50 ml) then evaporated to dryness (8.7 g). The residue was dissolved in boiling MeOH (120 ml) at room temperature, treated with charcoal (0.4 g), evaporated to a small volume. The solid was collected by filtration, washed with cold MeOH of compound **5.**

The alcoholic mother liquid was evaporated to dryness. The residue was treated with EtOH to give the second crop of compound **5.**

Yield:
First crop: 6.22 g (28.1%), Mp: 177.5-178.5°C
Second crop: 0.76 g (3.4%), Mp: 176.0-178.0°C
Total: 6.94 g (31.3%)

### Preparation of 1-Propargylaminoindan-3-one mesylate 7

1-propargylaminoindan-3-one hydrochloride 5 (11.1 g, 0.05 mole) was stirred in a mixture of CH₂Cl₂ (150 ml) and water (130 ml). 20% NaOH was added to reach pH 12. The phases then were separated. The aqueous phase was extracted with CH₂Cl₂ (50 ml).

The combined organic phase was extracted with a nearly saturated NaCl solution (50 ml). The organic phase was treated with Na₂SO₄ and charcoal (0.9 g). Methanesulfonic acid (4.81 g, 3.25 ml, 0.05 mole) in CH₂Cl₂ was added drop by drop to the stirred solution of the amine base. After an hour the solid was collected by filtration, washed with CH₂Cl₂, dried. The mesylate salt (13.7 g) was dissolved in water (44 ml) at 60°C, treated with charcoal (1 g). The solution was put into the fridge overnight.

The crystals were collected by filtration, washed with a little amount of cold water then EtOH. The mother liquid was evaporated to a small volume to give the second crop.

Yield:
First crop: 6.99 g (49.7%), Mp: 186-190°C
Second crop: 3.77 g (26.8%), Mp: 184-190°C
Total: 10.76 g (76.5%)

### Example 2: Pilot Scale Production of Rasagiline

### Step 1 - Preparation of Racemic PAI base, 1000 liter stainless steel (SS) reactor, air atmosphere

1-Aminoindan (40kg), toluene (131kg), soft water (152kg) and technical grade NaOH (28kg of 47% solution) were introduced into reactor at stirring and were mixed at an ambient temperature. 60kg of Propargyl Benzene Sulfonate (PBS) were added by portions over 45 min. and the reaction mixture was heated 40°C and was further held for 5 hrs at about 41-46°C. After completing the reaction, the stirring was stopped and the reaction mixture was settled at this temperature for 30 min. Lower aqueous phase was separated and discarded.

Upper organic phase was mixed with 120kg of soft water and 33% H₂SO₄ solution was added to the reaction mixture by portions. During the addition of 33% H₂SO₄ the reaction temperature was maintained within the range of 40-47°C and the pH of the mixture was controlled by a pH-meter.

After adjusting the pH to pH=2.0 (44kg of 33% H₂SO₄ solution added) the stirring was stopped and the batch was settled for 30 min. The phases were separated using a separation tank and an organic phase was discarded. The aqueous phase was re-introduced into the SS reactor.

Toluene (51kg) was added to the batch and then the pH was adjusted by addition of 25% NaOH at stirring to pH=6.3, temperature was maintained within the range of 40-47°C. The stirrer was stopped and the reaction mixture was settled at this temperature for 30 min. The phases were separated using a separation tank, organic phase was transferred to a drum (Extract I) and aqueous phase was re-introduced into the SS reactor.

Toluene (51kg) was added to the aqueous phase, the batch was stirred and then pH=7.0 was adjusted by addition of 25% NaOH, temperature was maintained within the range 40-47°C. The stirrer was stopped and the reaction mixture was settled at this temperature for 30 min. The aqueous phase was discarded and organic phase was mixed with the organic phase from previous extraction (Extract I) in the SS reactor.

The combined organic solution was washed with 150 liters of soft water at 40-47°C by stirring for 30 min. The stirring then was stopped and the mixture was settled at this temperature for additional 30 min. The phases were separated. The aqueous phase was discarded and the solvent of the organic phase was evaporated under vacuum by heating and stirring.

After the completion of Toluene evaporation, Isopropanol (47kg) was added to the residue and evaporated under the same conditions. The residue of evaporation (oil) was cooled to 30°C and transferred into container.

Product - 31.3 kg racemic PAI base, Assay - 89.3%

### Step 2 - Preparation of Rasagiline Tartrate, 250 liter SS reactor, SS centrifuge air atmosphere

Racemic PAI base (31.3kg as is) from Step 1 was introduced into a reactor with Isopropanol (64kg) and heated to reflux at stirring. A solution of 17kg of L-Tartaric acid in 17kg of soft water was introduced into boiling solution at 80°C over 30min. Isopropanol (10kg) was introduced to the batch through the reactor feed line. Reflux was maintained in the reactor for 55 min, crystallization of Rasagiline tartrate was observed.

The resulting slurry was cooled 25°C and stirred at this temperature for one hour. Then the batch was filtered in centrifuge and the cake was washed twice with Isopropanol (2 x 23kg). Then the wash was extracted from the cake by spinning and solid product transferred to a container. Filtrate was discarded to waste.

Product - 23.0kg of Rasagiline tartrate (wet).

Analysis:
S-isomer by HPLC: 1%
Impurities by HPLC:
1-Aminoindan 0.02%; 3-PAIO - 0% (N.D.); RRT=0.62 - 0.1%

### Step 3 - Preparation of Rasagiline Mesylate in air atmosphere

### Preparation of Rasagiline base:

Solid NaOH (C.P. pearls, 3.3kg) was dissolved in 55 liters of soft water in a 500 liter SS reactor at stirring. Wet Rasagiline tartrate (23.0kg) from Step 2 was introduced into the reactor and 55 liters of Toluene were added. The mixture then was heated to 43°C at stirring. After complete dissolution of the solid the batch was filtered into a glass lined (GL) 500 liter reactor through 10µ filter. The line and the filter were washed with additional 10 liters of Toluene.

The batch was settled in GL reactor at 40-50°C for 30 min. and the lower aqueous phase was separated and discarded.

The organic phase was washed with 27 liters of soft water at 40-50°C for 30 min. then settled at the same temperature for additional 30 min. The lower aqueous phase was separated and discarded. An organic phase was left in the reactor. The solvent was distilled from the organic phase under vacuum, then 27 liters of Isopropanol were introduced into the reactor and the distillation was repeated. Residue of evaporation (rasagiline base oil) was cooled to 30°C and mixed with 81 liters of Isopropanol.

### Preparation of Rasagiline Mesylate:

Methane Sulfonic Acid (MSA, 7.8kg) was added to the batch at cooling and stirring by portions during 10 min., precipitation of mesylate salt took place. Resulting suspension was heated to reflux and after complete dissolution of solids at reflux conditions the batch was filtered through 10µm filter to GL reactor-crystallizer (volume 200 liter). The crystallizer was heated to reflux at stirring and then cooled to temperature 10°C over a period of 5 hours. During the cooling crystallization of mesylate salt took place.

The batch was held at 8-10°C for 30 min. and was further filtered in SS centrifuge. Mother liquor was extracted and the filtrate was sampled and discarded. The cake was washed twice with Isopropanol (2 x 15 liter), the liquor was extracted from the cake by spinning and wet rasagiline mesylate (18.6kg) was transferred to a container.

### Processing of solid Rasagiline Mesylate:

Wet rasagiline mesylate (18.6kg) was introduced into SS Drier (Acinoxa 80 liter volume) and dried under vacuum at heating (jacket temperature 63°C) and agitation for 3hrs.

Dry rasagiline mesylate (15.9kg) was milled using SS cone mill (Comil).

Analysis of dry rasagiline mesylate:
Impurities by HPLC: 3-PAIO - 0.11% (Out of specification)

### Discussion of Example 2:

During the production of rasagiline drug substance under certain conditions, a by-product 3-keto-N-propargyl-1-aminoindan may be formed. Although 3-keto-N-propargyl-1-aminoind is not genotoxic, its presence affects purity of the pharmaceutical product and is therefore undesirable.

It has been proposed that components of metal equipment (specifically stainless steel equipment) and reaction under air atmosphere may promote oxidation of 3-propargyl aminoindan and subsequent formation of 3-keto-N-propargyl-1-aminoindan.

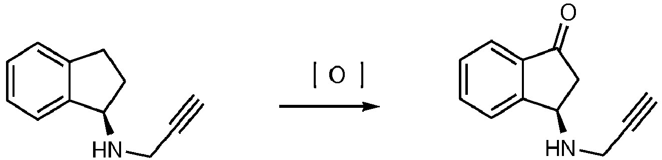

Indeed, it has been shown that switching the equipment from stainless steel to glass and performing the process under an inert atmosphere prevents formation of the impurity. It has been further observed that manufacture of rasagiline tartrate (a starting material) using stainless steel equipment leads to out of specification amounts of 3-keto-N-propargyl-1-aminoindan (0.11%) in the final product (rasagiline mesylate) even when the latter has been crystallized in a glass reactor. This implies that performing all the steps of the production process in a metal-free environment and under an inert atmosphere are required to prevent oxidation and formation of the undesirable impurity. It should be noted that rasagiline tartrate serves as an intermediate in the production of different rasagiline forms, including rasagiline mesylate, rasagiline free base and others. Therefore the above-identified precautions should be exercised during the manufacture of all rasagiline forms.

### Example 3: Commercial-scale production of Rasagiline Mesylate under inert atmosphere in non-metal conditions

### Step 1 - Preparation of racemic PAI base, 1200 liter glass-lined reactor, with PTFE lined piping and under nitrogen atmosphere.

1-Aminoindan (90kg), toluene (180kg), soft water (287kg) and pure NaOH (118kg of 25% solution) were introduced into reactor at stirring and were mixed at ambient temperature. Then 135kg of Propargyl Benzene Sulfonate (PBS) and 55kg of toluene were added by portions over 45 minutes and the reaction mixture was heated 40°C and held for 4½ hrs at 41-47°C. After the reaction completion the stirrer was stopped and the reaction mixture was settled at this temperature for 30 minutes. Lower aqueous phase was separated and discarded to waste.

Upper organic phase was mixed with 270kg of soft water and 33% H₂SO₄ solution was then added by portions. During the addition reaction temperature was maintained within the range 40-47°C and pH of the mixture was monitored by pH-meter.

After adjusting pH of reaction mixture to 2.4 (94kg of 33% H₂SO₄ solution added) the stirrer was stopped and the batch was settled for 30 minutes. The lower aqueous phase was separated using glass separation tank, organic phase was discarded to waste and the aqueous phase was re-introduced into the reactor.

Toluene (155kg) was added to the batch and then pH was adjusted to 6.1 by addition of 25% NaOH at stirring (82kg added) while temperature was maintained within the range 44-46°C. The stirrer was stopped and the reaction mixture was settled at this temperature for 30 minutes. Lower aqueous phase was separated using glass separation tank, organic phase was transferred to glass lined vessel (Extract I) and aqueous phase was re-introduced into the reactor.

Toluene (115kg) was added to the aqueous phase, the batch was stirred and then pH was adjusted to 6.7 by addition of 25% NaOH (3kg added) while temperature was maintained within the range 45-47°C. The stirrer was stopped and the reaction mixture was settled at this temperature for 30 minutes. Lower aqueous phase was separated to waste and organic phase was mixed with the organic phase from previous extraction (Extract I) held in glass lined vessel.

The combined organic solution was washed with 377 liters of soft water at 41-46°C by stirring for 1½ hours. Then the stirrer was stopped and the mixture was settled at this temperature for 30 minutes. Lower aqueous phase was separated to waste and organic phase was evaporated under vacuum at heating and stirring.

After completion of Toluene evaporation, Isopropanol (106kg) was added to the residue and evaporated under the same conditions.

The residue of evaporation (oil) was cooled to 30°C and transferred into glass lined vessel.

Product - 78kg racemic PAI base, Assay - 96.6%

### Step 2 - preparation of Rasagiline Tartrate, 600 liter glass lined reactor with PTFE lined piping, SS centrifuge, under nitrogen atmosphere

Racemic PAI base (78kg as is) prepared in Step 1 was introduced into reactor with Isopropanol (172kg) and heated to reflux at stirring.

Solution of 64kg of L-Tartaric acid solution in soft water (39 wt%) was introduced into boiling solution at 80°C over 30 minutes. Isopropanol (10kg) was introduced to the batch through the reactor feed line.

Reflux was maintained in the reactor for 1½ hours and crystallization of Rasagiline Tartrate was observed.

The resulting slurry was cooled to 25°C and was stirred at this temperature for one hour. Then the batch was filtered in centrifuge and the cake was washed with Isopropanol (3x32kg). The wash was extracted from the cake by spinning and solid product was transferred to container. Filtrate was discarded to waste.

Product - 69.9kg of Rasagiline Tartrate (wet)

Analysis:
S-isomer by HPLC - Less Than (L.T.) 4%
Impurities by HPLC: 1-Aminoindan - L.T. 0.08%, 3-PAIO - L.T. 0.02%

### Step 3 - preparation of Rasagiline Mesylate, 1200 liter, 600 liter and 300 liter glass lined reactors with PTFE lined piping, SS centrifuge, SS dryer, SS mill, under nitrogen atmosphere

### Preparation of Rasagiline base:

25% NaOH solution (37kg), deionized water (109kg), wet Rasagiline Tartrate (69.9kg) prepared in Step 2 were introduced into 1200 liter glass lined reactor. Then 135 kg of Toluene was added and the mixture was heated to 42°C at stirring. After complete dissolution of solid the batch was filtered from the 1200 liter reactor to 600 liter glass lined reactor through 10µ filter, the line and the filtered were washed with additional 50 liters of Toluene.

The batch was settled in 6001 reactor at 40-50°C for 30 minutes and the lower aqueous phase was separated and discarded to waste.

The organic phase was washed with 65kg of soft water at 40-50°C for 30 minutes and then was settled at the same temperature for 30 minutes. The lower aqueous phase was separated and discarded to waste and the organic phase remained in the reactor.

The solvent was distilled from the organic phase under vacuum, then 80kg of Isopropanol was introduced into the reactor and the distillation was repeated.

Residue of evaporation (Rasagiline base oil) was cooled to 30°C and mixed with 187kg of Isopropanol.

### Preparation of Rasagiline Mesylate:

Methane Sulfonic Acid (MSA, 21.1kg) was added to the batch at cooling and stirring by portions over 10 minutes. Precipitation of mesylate salt took place.

Resulting suspension was heated to reflux and after complete dissolution of solids at reflux conditions the batch was filtered through 10µ filter to GL reactor-crystallizer (volume 300 liter). The filter was washed with 27kg isopropanol, then the crystallizer was heated to reflux at stirring and cooled to temperature 10°C over a period of 5 hours. During the cooling crystallization of mesylate salt took place.

The batch was held at 8-10°C for 3½ hours and was filtered in SS centrifuge under nitrogen atmosphere. Mother liquor was extracted the filtrate was sampled and discarded to waste. The cake was washed twice with Isopropanol (2x30liter). The liquor was extracted from the cake by spinning and wet Rasagiline Mesylate (52.3kg) was transferred to container.

### Processing of solid Rasagiline Mesylate:

Wet Rasagiline Mesylate (52.3kg) was introduced into SS Drier ("Charles Thompson", 100liter volume) and dried under vacuum at heating (jacket temperature 63°C) and agitation for 3 hours.

Dry Rasagiline Mesylate (44.3kg) was milled using SS cone mill (Comil).

Analysis of Dry Rasagiline Mesylate:
Impurities by HPLC: 3-PAIO - 0.01% (conforms to the specification level of L.T. 0.1%)

### Example 4: Pilot-scale production of (Rasagiline Base under inert atmosphere in non-metal conditions

### Step 1 - Purification of Rasagiline Tartrate, 60 liter glass lined reactor with PTFE piping, Hastelloy filter - dryer, under nitrogen atmosphere

Wet Rasagiline Tartrate (10kg) prepared as described in the Steps 1 and 2 of the Example 3 was introduced with 28kg of process water into 60 liter glass lined reactor. The mixture was heated to 75°C at stirring and held at 75-80°C for 1½ hours. Then the batch was cooled to 30°C and 10.2kg isopropanol was added. The batch was cooled to 10°C and was stirred at this temperature for 30 minutes.

The suspension was transferred to filter-dryer and was filtered under pressure of nitrogen. The cake was washed 3 times with isopropanol (3×1.8kg) under nitrogen and dried.

The drying was performed at 55°C under vacuum with cake agitation over 14 hours.

6.0kg of Dry Pure Rasagiline Tartrate was obtained.

### Step 2 - Preparation of Rasagiline Base, 30 and 60 liter glass lined reactors with PTFE piping, Hastelloy filter - dryer, under nitrogen atmosphere

25% NaOH solution (5.8kg), deionized water (13.2kg), dry Pure Rasagiline Tartrate (6.0kg) prepared in Step 1 was introduced into 60 liter glass lined reactor. 13 kg of Toluene (13kg) was added and the mixture was heated to 40°C at stirring. After complete dissolution of solid the batch was stirred at 40-47°C for 30 minutes then settled without stirring at the same temperature for phase separation.

Lower aqueous phase was separated and discarded. Organic phase was washed in the reactor with 8kg process water at 44-47°C.

The batch was settled in reactor at 47-49°C for 30 minutes and the lower aqueous phase was separated and discarded to waste, organic phase remained in the reactor.

The solvent was distilled from the organic phase under vacuum, then 6.1kg of ethanol were introduced into the reactor and the distillation was repeated.

Residue of evaporation (Rasagiline base oil) was cooled to 19°C and mixed with 2.6kg of absolute ethanol. The solution was transferred through 0.2µ filter to 30 liter glass lined reactor. The line and the filter were washed with 1.9kg absolute ethanol.

Combined ethanolic solution and wash were cooled to 11°C at stirring and 2 kg of process water added to the batch.

Cooling was continued, crystallization of Rasagiline base started and batch was stirred at 11-12°C for 1¼ hours. Then 8.5kg of process water was added by portions during one hour.

The batch was cooled to 6°C and was held at this temperature for 30 minutes and then transferred to filter-dryer. The solid product was filtered under pressure of nitrogen and was washed twice with process water in nitrogen atmosphere.

The cake was dried under vacuum at agitation and gentle heating (jacket temperature 35°C) for 19 hours.

Dry product - 3.7kg
Analysis of Dry Rasagiline Base:
Impurities by HPLC: 3-PAIO - L.T. 0.02% (conforms to the specification level of L.T. 0.1%)

### Discussion of Examples 3 and 4:

The data presented in Examples 3 and 4 demonstrate that commercial-scale production of Rasagiline Mesylate and Rasagiline Base Drug Substances with very low level of 3-PAIO (L.T. 0.02%) can be prepared under non-metal synthesis conditions and under inert atmosphere (nitrogen).

The invention may be characterised by the following clauses;
1. A composition comprising N-propargyl-1(R)-aminoindan or a pharmaceutically acceptable salt thereof, and 3-keto-N-propargyl-1-aminoind or a salt thereof, wherein the total amount of 3-keto-N-propargyl-1-aminoindan which is present in the composition is less than 0.10% relative to the amount of N-propargyl-1(R)-aminoindan, based on a determination by an HPLC method.
2. The composition of clause 1, wherein the total amount of 3-keto-N-propargyl-1-aminoindan which is present in the composition is greater than 0.02% relative to the amount of N-propargyl-1(R)-aminoindan.
3. The composition of clause 1, wherein the total amount of 3-keto-N-propargyl-1-aminoindan which is present in the composition is greater than 0.05% relative to the amount of N-propargyl-1(R)-aminoindan.
4. The composition of clause 1, wherein the total amount of 3-keto-N-propargyl-1-aminoindan which is present in the composition is less than 0.05% relative to the amount of N-propargyl-1(R)-aminoindan.
5. The composition of clause 1, wherein the total amount of 3-keto-N-propargyl-1-aminoindan which is present in the composition is less than 0.02% relative to the amount of N-propargyl-1(R)-aminoindan.
6. The composition of any one of clauses 1-5, wherein the pharmaceutically acceptable salt of N-propargyl-1(R)-aminoindan is a mesylate salt.
7. The composition of any one of clauses 1-5, wherein the pharmaceutically acceptable salt of N-propargyl-1(R)-aminoindan is a citrate salt.
8. The composition of any one of clauses 1-5, wherein N-propargyl-1(R)-aminoindan is present in the form of a free base.
9. The composition of any one of clauses 1-8, further comprising at least one pharmaceutically acceptable carrier.
10. The composition of clause 9, wherein the pharmaceutically acceptable carrier is selected from the group consisting of mannitol, starch, pregelatinized starch, colloidal silicon dioxide, stearic acid and talc.
11. The composition of any one of clauses 1-10, wherein the 3-keto-N-propargyl-1-aminoindan is 3-keto-N-propargyl-1(R)-aminoindan.
12. A process for the manufacture of a composition comprising N-propargyl-1(R)-aminoindan or a pharmaceutically acceptable salt thereof, comprising producing dry rasagiline tartrate from racemic propargyl aminoindan in metal-free equipment, and producing the composition.
13. The process of clause 12, wherein the step of producing dry rasagiline tartrate from racemic propargyl aminoindan is performed under an inert atmosphere.
14. The process of clause 12 or 13, wherein the pharmaceutically acceptable salt of N-propargyl-1(R)-aminoindan is a mesylate salt.
15. The process of clause 12 or 13, wherein the pharmaceutically acceptable salt of N-propargyl-1(R)-aminoindan is a citrate salt.
16. The process of clause 12 or 13, wherein N-propargyl-1(R)-aminoindan is present in the form of a free base.
17. The process of any one of claims 13-16, wherein the inert atmosphere is a nitrogen atmosphere.
18. The process of any one of clauses 12-17, wherein the metal-free equipment is glassware equipment.
19. A process for preparing a pharmaceutical product comprising N-propargyl-1(R) aminoindan or a pharmaceutically acceptable salt thereof, and at least one pharmaceutically acceptable carrier, comprising:
   a) obtaining a batch of N-propargyl-1(R)-aminoindan or a pharmaceutically acceptable salt thereof;
   b) determining the total amount of 3-keto-N-propargyl-1-aminoind in the batch; and
   c) preparing the pharmaceutical product from the batch only if the batch is determined to have less than 0.10% 3-keto-N-propargyl-1-aminoind relative to N-propargyl-1(R)-aminoindan, based on a determination by an HPLC method.
20. The process of clause 19, wherein in step c) the pharmaceutical product is prepared from the batch only if the batch is determined to have 3-keto-N-propargyl-1-aminoindan present in an amount of less than 0.10% relative to N-propargyl-1(R)-aminoindan.
21. The process of clause 19 or 20, wherein the pharmaceutical product is prepared from the batch only if the batch is determined to have less than 0.05% 3-keto-N-propargyl-1-aminoindan relative to N-propargyl-1(R)-aminoindan.
22. The process of clause 19 or 20, wherein the pharmaceutical product is prepared from the batch only if the batch is determined to have less than 0.02% 3-keto-N-propargyl-1-aminoindan relative to N-propargyl-1(R)-aminoindan.
23. The process of clause 20, wherein the pharmaceutical product is prepared from the batch if the batch is determined to have 3-keto-N-propargyl-1-aminoind present in an amount of greater than 0.05% relative to N-propargyl-1(R)-aminoindan.
24. The process of clause 20, wherein the pharmaceutical product is prepared from the batch if the batch is determined to have 3-keto-N-propargyl-1-aminoind present in an amount of greater than 0.02% relative to N-propargyl-1-aminoindan.
25. The process any one of clauses 19-24, wherein the pharmaceutically acceptable salt of N-propargyl-1(R)-aminoindan is a mesylate salt.
26. The process of any one of clauses 19-24, wherein the pharmaceutically acceptable salt of N-propargyl-1(R)-aminoindan is a citrate salt.
27. The process of any one of clauses 19-24, wherein N-propargyl-1(R)-aminoindan is present in the form of a free base.
28. A process of distributing a validated batch of a pharmaceutical product comprising N-propargyl-1(R)-aminoindan or a pharmaceutically acceptable salt thereof and at least one pharmaceutically acceptable carrier, comprising:
   a) producing a batch of the pharmaceutical product;
   b) performing stability testing with a sample of the batch;
   c) determining the total amount of 3-keto-N-propargyl-1-aminoind in the sample of the batch after stability testing; and
   d) validating the batch for distribution only if the sample of the batch after stability testing is determined to have less than 0.10% of 3-keto-N-propargyl-1-aminoindan relative to N-propargyl-1(R)-aminoindan, based on a determination by an HPLC method.
29. The process of clause 28, wherein in step d) the batch is validated only if the sample of the batch after stability testing is determined to have 3-keto-N-propargyl-1-aminoindan present in an amount of less than 0.10% of relative to N-propargyl-1(R)-aminoindan.
30. The process of clause 28 or 29, wherein the batch is validated only if the sample of the batch after the stability testing is determined to have less than 0.05% 3-keto-N-propargyl-1-aminoindan relative to N-propargyl-1(R)-aminoindan.
31. The process of clause 28 or 29, wherein the batch is validated only if the sample of the batch after the stability testing is determined to have less than 0.02% 3-keto-N-propargyl-1-aminoindan relative to N-propargyl-1(R)-aminoindan.
32. The process of clause 29, wherein the batch is validated if the sample of the batch after the stability testing is determined to have 3-keto-N-propargyl-1-aminoind present in an amount of greater than 0.02% of relative to N-propargyl-1(R)-aminoindan.
33. The process of clause 29, wherein the batch is validated if the sample of the batch after the stability testing is determined to have 3-keto-N-propargyl-1-aminoind present in an amount of greater than 0.05% of relative to N-propargyl-1(R)-aminoindan.
34. The process of any one of clauses 28-33, wherein the pharmaceutically acceptable salt of N-propargyl-1(R)-aminoindan is a mesylate salt.
35. The process of any one of clauses 28-33, wherein the pharmaceutically acceptable salt of N-propargyl-1(R)-aminoindan is a citrate salt.
36. The process of any one of clauses 28-33, wherein N-propargyl-1(R)-aminoindan is present in the form of a free base.
37. The process of any one of clauses 19-36, wherein the pharmaceutically acceptable carrier is selected from the group consisting of mannitol, starch, pregelatinized starch, colloidal silicon dioxide, stearic acid and talc.
38. The process of any one of clauses 12-37, wherein 3-keto-N-propargyl-1-aminoind is 3-keto-N-propargyl-1 (R)-aminoindan.
39. An isolated compound having the structure: 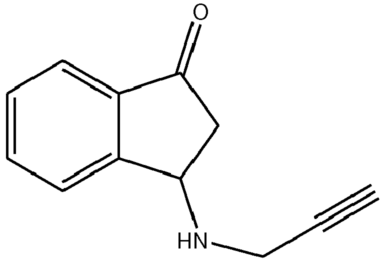 or a salt thereof.
40. The isolated compound of clause 39, having the structure: 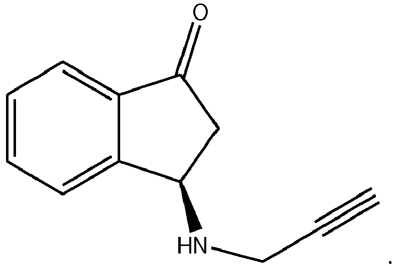
41. The isolated compound of clause 39, having the structure: 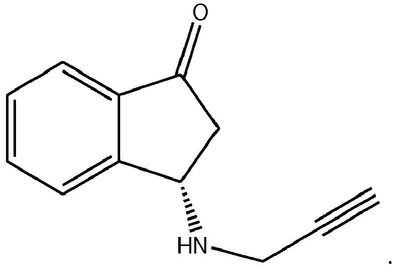
42. A composition comprising a compound having the structure: 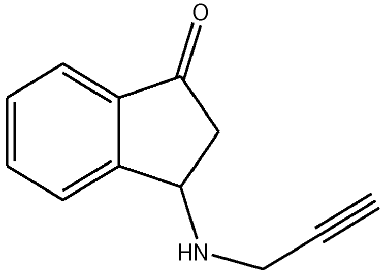 wherein the composition is free of N-propargyl-1-aminoindan or a salt thereof.
43. The composition of clause 42, wherein the compound has the structure: 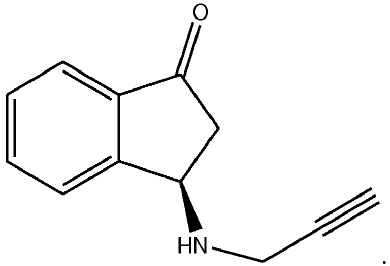
44. The composition of clause 42, wherein the compound has the structure: 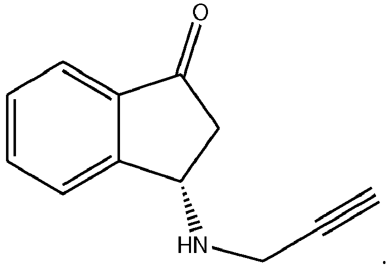
45. A process for the manufacture of 3-keto-N-propargyl-1-aminoinda or an enantiomer or a salt thereof, comprising reacting 1-aminoindane-3-one with a propargylating agent in the presence of a base so as to produce the compound.
46. The process of clause 45 further comprising a step of purifying the 3-keto-N-propargyl-1 (R)-aminoindan enantiomer.
47. The process of clause 45 or 46, wherein 1-aminoindane-3-one is in the form of a hydrochloride salt.
48. The process of any one of clauses 45-47, wherein the propargylating agent is selected from the group consisting of propargyl-bromide, propargyl-chloride, propargyl-iodide and propargyl alkyl sulfonate.
49. Use of 3-keto-N-propargyl-1-aminoinda or an enantiomer or a salt thereof, as a reference standard to detect trace amounts of impurities in a pharmaceutical product comprising N-propargyl-1(R)-aminoind or a pharmaceutically acceptable salt thereof.
50. The use of clause 49, wherein the enantiomer of 3-keto-N-propargyl-1-aminoind is 3-keto-N-propargyl-1 (R)-aminoindan.
51. The use of clause 49 or 50, wherein the impurity is a by-product.

## Claims

1. A composition comprising N-propargyl-1(R)-aminoindan or a pharmaceutically acceptable salt thereof, and 3-keto-N-propargyl-1-aminoind or a salt thereof, wherein the total amount of 3-keto-N-propargyl-1-aminoindan which is present in the composition is greater than 0.02% relative to the amount of N-propargyl-1(R)-aminoindan and is less than 0.10% relative to the amount of N-propargyl-1(R)-aminoindan, based on a determination by an HPLC method.

2. The composition of claim 1, wherein the total amount of 3-keto-N-propargyl-1-aminoindan which is present in the composition is greater than 0.05% relative to the amount of N-propargyl- 1(R)-aminoindan.

3. The composition of claim 1 or 2, wherein the pharmaceutically acceptable salt of N-propargyl-1(R)-aminoindan is a mesylate salt or a citrate salt.

4. The pharmaceutical composition of claim 1 or 2, wherein the pharmaceutically acceptable salt of N-propargyl-1(R)-aminoindan is a tartrate salt.

5. The pharmaceutical composition of any one of claim 1-3, wherein the pharmaceutically acceptable salt of N-propargyl-1(R)-aminoindan is a mesylate salt.

6. The composition of any one of claims 1-5, wherein N-propargyl-1(R)-aminoindan is present in the form of a free base.

7. The composition of any one of claims 1-6, further comprising at least one pharmaceutically acceptable carrier.

8. The composition of any one of claims 1-7, wherein the 3-keto-N-propargyl-1-aminoindan is 3-keto-N-propargyl-1(R)-aminoindan.

9. A process for the manufacture of a composition comprising N-propargyl-1(R)-aminoindan or a pharmaceutically acceptable salt thereof, comprising producing dry rasagiline tartrate from racemic propargyl aminoindan in metal-free equipment, which is other than a round-bottom flask, and producing the composition.

10. A process for preparing a pharmaceutical product comprising N-propargyl-1(R)-aminoindan or a pharmaceutically acceptable salt thereof, and at least one pharmaceutically acceptable carrier, comprising:
a) obtaining a batch of N-propargyl-1(R)-aminoindan or a pharmaceutically acceptable salt thereof;
b) determining the total amount of 3-keto-N-propargyl-1-aminoind in the batch; and
c) preparing the pharmaceutical product from the batch only if the batch is determined to have less than 0.10% 3-keto-N-propargyl-1-aminoind relative to N-propargyl-1(R)-aminoindan, based on a determination by an HPLC method.

11. A process of distributing a validated batch of a pharmaceutical product comprising N-propargyl-1(R)-aminoindan or a pharmaceutically acceptable salt thereof and at least one pharmaceutically acceptable carrier, comprising:
a) producing a batch of the pharmaceutical product;
b) performing stability testing with a sample of the batch;
c) determining the total amount of 3-keto-N-propargyl-1-aminoind in the sample of the batch after stability testing; and
d) validating the batch for distribution only if the sample of the batch after stability testing is determined to have less than 0.10% of 3-keto-N-propargyl-1-aminoindan relative to N-propargyl-1(R)-aminoinda based on a determination by an HPLC method.

12. The process of any one of claims 9-11, wherein the pharmaceutically acceptable salt is a tartrate salt.

13. The process of any one of claims 9-11, wherein the pharmaceutically acceptable salt is a mesylate salt.
